# EUROPEAN PATENT SPECIFICATION

(11) **EP 0 754 018 B1**
(45) Date of publication and mention of the grant of the patent: **22.05.2002**
(21) Application number: 94923190.6
(22) Date of filing: 10.06.1994
(51) Int. Cl.: A61F 2/44

(54) **INTERVERTEBRAL DISK ARTHROPLASTY DEVICE**
ARTHROPLASTISCHE VORRICHTUNG ALS BANDSCHEIBENERSATZ
DISPOSITIF D'ARTHROPLASTIE DESTINE AU REMPLACEMENT DES DISQUES INTERVERTEBRAUX

(30) Priority: 05.04.1994 US 223119
(43) Date of publication of application: 22.01.1997
(60) Divisional application: 01204312.1
(73) Proprietor: SDGI Holdings, Inc., Wilmington, DE 19801 (US)
(72) Inventor: BOYD, Lawrence, M., Memphis, TN 38117 (US); SALIB, Richard, M., Excelsior, MN 55331 (US); PETTINE, Kenneth, A., Fort Collins, CO 80525 (US)
(74) Representative: Allman, Peter John
(86) International application number: PCT/US94/06530
(87) International publication number: WO 95/26697

(56) References cited:
- EP-A- 0 566 810
- WO-A-91/13598
- WO-A-93/10725
- WO-A-94/04100
- DE-A- 3 023 353
- US-A- 4 349 921
- US-A- 5 024 670
- US-A- 5 258 031
- US-A- 5 314 477
- US-A- 5 314 478

## Description

### BACKGROUND OF THE INVENTION

The present invention is directed to a surgical joint replacement device; particularly, a replacement for a degenerated or ruptured disk between consecutive vertebrae in the spine.

Currently there are approximately 60,000 lumbar spine fusions performed in the United States and 30,000 lumbar fusions performed in Canada each year. Spinal fusion is frequently used as a treatment for low back pain and intervertebral disk degeneration, and the use of internal fixation has increased the ability of a surgeon to obtain a solid fusion. There is increased concern, however, that the biomechanical rigidity of the fusion and internal fixation may predispose adjacent spinal motion segments to rapid deterioration. Long-term follow up of patients undergoing a successful fusion indicates that 50 percent will continue to have complaints of pain. As in other joints, alternatives to fusing a spinal motion segment have inherent advantages.

Researchers have attempted to design a successful intervertebral disk arthroplasty device for years. U.S. Patent 4,946,378 discloses an artificial disk having a pair of end bodies with a medical synthetic polymeric intermediate member held between the end bodies. The intermediate member apparently provides some flexibility. Somewhat similarly, U.S. Patent 5,002,576 discloses an artificial disk having end cover plates separated by a closed corrugated tube which is filled with a viscoelastic material, like a body-compatible silicone. Also, U.S. Patent No. 4,932,975 shows a vertebral prosthesis having a pair of end members that house suspension plates surrounded by an elastomeric medium. The end housings are interconnected with flexible and expandable bellows.

Other approaches are shown in U.S. Patent Nos. 4,349,921, 4,714,469, 4,759,769, 4,863,476, 4,936,848, 4,997,432, 5,047,055, 5,071,437 and PCT Patent WO 92/14423. U.S. Patent No. 4,349,921 discloses an artificial disk-having convex superior and inferior surfaces corresponding to the adjacent vertebrae surfaces and being formed from two components to allow flexion and extension between the two components. U.S. Patent No. 4,714,469 discloses a single member artificial disk having a predetermined thickness. U.S. Patent No. 4,759,769 discloses an artificial disk having upper and lower members hinged together at a rear portion and biased apart at a front portion by stiff coil springs. U.S. Patent No. 4,863,476 discloses a two portion spinal implant that is expandable so as to increase the spacing between the adjacent vertebrae. U.S. Patent No. 4,936,848 shows an artificial disk having a spherical shape that is hollow and rigid. The sphere wall contains fenestrations, open to the sphere cavity, for placing bone fragments therein. U.S. Patent No. 4,997,432 shows an artificial disk having plates separated by a sliding core body normally consisting of a synthetic material. U.S. Patent No. 5,047,055 discloses an artificial disk made from a hydrogel material having a specified compressive strength and, when hydrated, having the shape of a human disk. U.S. Patent No. 5,071,437 shows an artificial disk having two rigid end-plates separated by, and connected to, an elastomeric core material having flexure properties similar to those of a human disk.

U.S. Patents 4,595,663, Re. 32,449 and 5,037,438 disclose the use of ceramic material, including zirconia, for applications such a joint replacement.

WO-A-94/04100 describes an intervertebral disk prosthesis having bone engaging plates provided with a central ball joint and having an intercalary cushioning ring between the plates and around the ball joint, which is offset rearwardly in the posterior part of the plates.

There are certain basic criteria a successful intervertebral disk arthroplasty device must fulfill. Fatigue strength of the materials is of utmost importance.

Since the average age of patients undergoing spinal fusion is 42 years old, the life span of the device should exceed 40 years. Assuming the average person experiences 2 million strides per year and 125,000 significant bends in the spine, a conservative estimate of the number of spinal loading cycles over the 40-year period would be 85 million cycles. To provide a factor of safety, the device should be designed to at least a fatigue limit of 100 million cycles.

In addition to such durability, the materials for a successful intervertebral disk arthroplasty device must be biocompatible. The amount of wear of the implant must be kept to a minimum. Although the implant should be small enough to be contained within the anatomic confines of a normal disk space, it is recognized that it may be advantageous to increase the prosthetic disk height in order to over distract the disk space to unload the facet joints posteriorly.

The present invention not only satisfies these criteria, but it is anticipated that it could be a successful arthroplasty in place of 90 per cent of the fusions currently being performed.

### SUMMARY OF THE INVENTION

The present invention is directed to an intervertebral disk arthroplasty device adapted to replace a disk between a first vertebra and a second vertebra in a spine, said arthroplasty device comprising:
a first member having a first joint surface, a top surface opposite said first joint surface, a first posterior end and a first anterior end;
a second member having a second joint surface facing said first joint surface, a bottom surface opposite said second joint surface, and second posterior and anterior ends juxtaposed with corresponding ones of said first posterior and anterior ends;
a first insert cup having a first receiving surface for receiving said top surface therein, and a first engaging surface opposite said first receiving surface for engaging the first vertebra; and a second insert cup having a second receiving surface for receiving said bottom surface therein, and a second engaging surface opposite said second receiving surface for engaging the second vertebra; characterised in that said first and second engaging surfaces are convex about the sagittal plane of the spine.
   Preferably, means are provided for restrainably retaining said top and bottom surfaces within said first and second receiving surfaces respectively.

The insert cups are preferably made of titanium. The use of titanium insert cups offers several advantages, including:
(1) providing tensile support to the structure and maintaining the first and second member material in compression;
(2) providing for the attachment of resisting fins to the anterior ends of the outer surface of an insert cup to further resist axial rotation by known processes such as machining, soldering, welding or gluing;
(3) providing a surface that coatings may easily adhere to, such as a titanium bead coating for promoting bony ingrowth fixation using known technologies such as sintering or spraying; and
(4) providing a structure wherein the first and second members can remain a single size while the insert cup sizes may be more easily and cheaply varied by either adding or reducing metal thickness.

The invention also contemplates instances where initial screw fixation may not be necessary. It has been shown that bone ingrowth can occur in ceramic. Thus, in one aspect of the invention, a preferred material for the first and second members is zirconium oxide ceramic or aluminum oxide ceramic. The indicated ceramic is biocompatible and the fatigue strength is expected to provide the necessary number of design cycles for the indicated use. Wear would be minimal and the implant can be made small enough to fit within the anatomic confines of a normal disk space. Although zirconium oxide ceramic or aluminum oxide ceramic are presently preferred, it is understood that other materials may be found which will also satisfy performance needs.

In any case, with material criteria satisfied, the invention then further provides for movement with up to 3 degrees of freedom mimicking normal intervertebral disk movement, except for compression. In one preferred embodiment, the disk arthroplasty device is shaped to provide up to 15 degrees of flexion, 5 degrees of extension 5 degrees of lateral bending, while restricting anterior/posterior shear, axial rotation and axial compression movements. In another embodiment, the device is shaped to provide the 3 degrees of movement described above and to provide unlimited axial rotation. The inventive arthroplasty device is designed to be non-compressible in order to maintain distraction of the facet joints posteriorly, thereby reducing the likelihood of a source of possible pain.

The arthroplasty device is placed in a distracted disk space so that the surrounding soft-tissue, ie. disc annulus, is in tension, to prevent the arthroplasty device from dislocating. In this environment, the ball and socket portions as defined by the invention eliminate any loose moving parts, while still providing for the greatest flexibility of movement available.

Thus, the advantages and objects obtained by the present invention are many. Further explanation and understanding is available by reference to the drawings briefly described hereinafter and to the detailed description thereafter.

### BRIEF DESCRIPTION OF THE DRAWINGS

FIG. 1 is a front representation of a lumbar spine showing healthy disks between vertebral bodies.

FIG. 2 is similar to FIG. 1, except a disk has been replaced by a prior art disk arthroplasty device.

FIG. 3 is a side cross-sectional view of a prior art disk arthroplasty device.

FIG. 4 is a front view of the device of FIG. 3.

FIG. 5 is a perspective view of a disk arthroplasty device in accordance with the present invention with the ball and socket configuration shown in phantom.

FIG. 6 is an anterior view of two vertebrae showing replacement of a human disk by a disk arthroplasty device in accordance with another embodiment of the present invention.

FIG. 7 is a side view of FIG. 5.

FIG. 8 is a perspective view of the lower member of a disk arthroplasty device in accordance with a further embodiment of the invention.

FIG. 9 is a perspective view of the upper member of a disk arthroplasty device with a socket configuration corresponding to the ball configuration of FIG. 8 shown in phantom.

FIG. 10 is a perspective view of the lower member of a disk arthroplasty device in accordance with a further embodiment of the invention with the ball configuration shown in phantom.

FIG. 11 is a perspective view of a disk arthroplasty device with a socket configuration corresponding to the ball configuration of FIG. 10.

FIG. 12 shows a top elevational view of a disk arthroplasty device with another ball configuration according to the present invention.

FIG. 13 is a cross-section of the disk arthroplasty device of FIG. 12 along section lines 13-13.

FIG. 14 is a cross-section of the disk arthroplasty device of FIG. 12 along section lines 14-14.

FIG. 15 is a top perspective view of an insert cup according to the present invention.

FIG. 16 is a side elevational view of the insert cup of FIG. 15.

FIG. 17 is a side elevational view of the insert cup of FIG. 15 with the lower member of a disk arthroplasty device mounted therein.

FIG. 18 is a top perspective view of another insert cup according to the present invention.

FIG. 19 is a side elevational view of the insert cup of FIG. 18.

FIG. 20 is a cross sectional view of a disk arthroplasty device mounted within a pair of insert cups of FIG. 18 and showing a screw attachment of the arthroplasty device to one of the insert cups.

FIG. 21 is an enlarged version of the circled area of FIG. 20 showing a screw attachment of a disk arthroplasty member to the insert cup of FIG. 18.

FIG. 22 shows a top perspective view of an insert cup incorporating rows of resisting teeth and a bead pocket.

### DESCRIPTION OF THE PREFERRED EMBODIMENT

For the purposes of promoting an understanding of the principles of the invention, reference will now be made to the embodiment illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

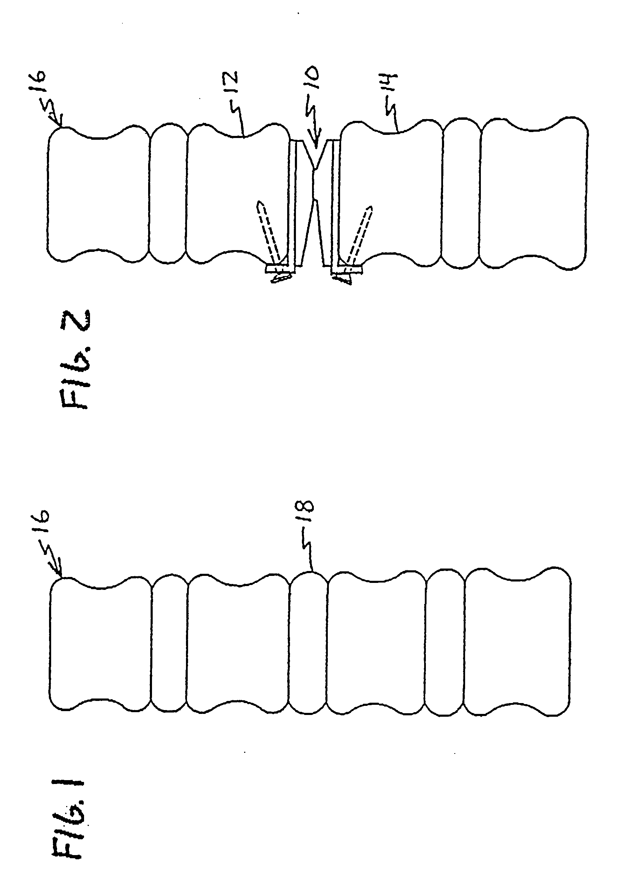

Referring now to FIGS. 1 - 4 wherein like reference numerals designate identical or corresponding parts throughout the several views, and more particularly to FIG. 2, a prior art intervertebral disk arthroplasty device is designated generally by the numeral 10. Device 10 is shown implanted between a first vertebral body 12 above it and a second vertebral body 14 below it, all being part of a representative lumbar spinal column 16. Lumbar spine 16 is shown in FIG. 1 to include a healthy disk 18. Device 10 is a prosthetic disk replacement in the space created by resected disk 18.

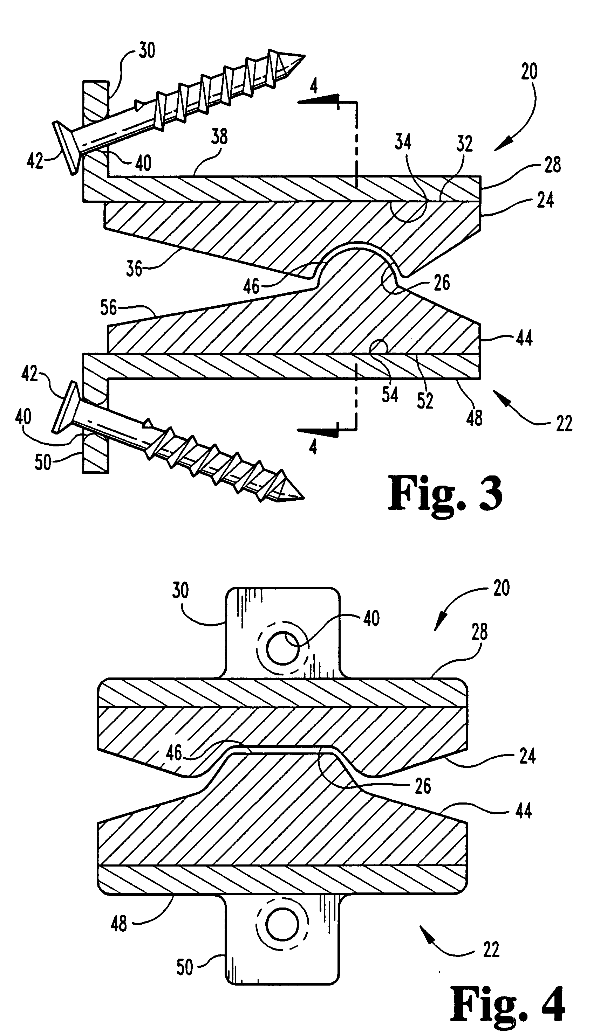

As shown in FIGS. 3 and 4, disk arthroplasty device 10 includes an upper assembly 20 and a lower assembly 22. Upper assembly 20 includes a first member 24 having a socket 26 therein. A base plate 28 having an upwardly extending tab 30 therefrom is fastened to first member 24. First member 24 has an upper side 32 to conform with and be fastened to an adjacent side 34 of base plate 28. A side 36 opposite upper side 32 includes socket 26 formed therein. Side 36 is formed so that it slopes upwardly on all sides of socket 26 such that the sloping is away from the entrance to socket 26 and toward upper side 32.

Base plate 28 is essentially a flat plate fastened with a biocompatible adhesive or other fastening mechanism known to those skilled in the art along side 34 to side 32 of first member 24. The upper side 38 of base plate 28 is formed to conform with vertebral body 12. Generally, side 38 is flat, and the conforming side of vertebral body 12 is made flat during the surgery. Tab 30 extends upwardly and is integral with base plate 28. Tab 30 includes an opening 40 therethrough so that a screw 42 (shown with respect to lower base plate 48) can be used to fasten assembly 20 to vertebral body 12.

Lower assembly 22 includes a second member 44 having a ball 46 formed thereon which fits loosely into socket 26. Base plate 48 having tab 50 is similar to upper base plate 28 with tab 30 and need not be described further. First member 44 has a lower side 52 which is fastened to an adjacent side 54 of base plate 48 and in a similar fashion as earlier described with respect to first member 24 and upper base plate 28. A side 56 opposite from lower side 52 includes ball 46 extending upwardly therefrom. Side 56 slopes away from ball 46 on all sides of ball 46 so as to create space between first and second members 24, 44 except where they fit together at ball 46 and socket 26. In one preferred embodiment, sides 56 and 36 slope away from ball 46 and socket 26 along an inclined plane in four different directions. Ball 46 and socket 26 generally have an oval shape or an elongated shape with quarter spherical shapes at the ends.

A motion segment with respect to the present invention comprises a disk arthroplasty device 10 and adjacent upper and lower vertebral bodies. The exact contours of ball 46 and socket 26 and and the surrounding surfaces of sides 36 and 56 determine the range of motion allowed in flexion and extension, side bending, shear and rotation of the motion segment.

The primary motion observed in a healthy lumbar intervertebral joint is flexion-extension. A typical L4-L5 intervertebral disk allows 13 degrees of flexion, 3 degrees of extension, 3 degrees of lateral side bending, 1 degree of axial rotation, and a small amount of shear. The center of rotation for flexion-extension is located in the posterior portion of the intervertebral disk space. The present invention is based on a concept of loose constraint. One preferred embodiment allows 3 degrees of freedom mimicking the normal intervertebral disk movements of flexion, extension and lateral bending. Axial rotation, anterior/posterior shear and axial compression are restricted by disk arthroplasty device 10 in order to protect the posterior facet joints. In addition, it is inserted by distracting the disk space which will place the surrounding soft tissue constraints in tension. This helps prevent the arthroplasty device from dislocating. The device is shaped to provide 15 degrees of flexion, 5 degrees of extension, 5 degrees of side bending, 5 degrees of rotation, and 2 millimeters of shear. The articulating surfaces are the concave female socket surface which articulates with the male concave ball surface. Such design eliminates loose moving parts.

The present invention contemplates the use of a material for the first and second members 24 and 44 that will result in low particulate generation and that will prevent axial movement in compression. This application appears to be well suited for ceramic materials and one preferred embodiment uses zirconium oxide as the ceramic material while another preferred embodiment uses aluminum oxide. The material specifications show ideal wear characteristics and biocompatibility. The modulus of elasticity is less than previously available ceramics, and is less prone to cracking. It would appear that the material would last the required 40 years as a replacement disk arthroplasty.

The prior art device shown in FIGS. 1 - 4 has the ceramic members 24 and 44 fastened to base plates made preferably from either chrome cobalt or titanium. The purpose for the metal base plates is to enhance bone ingrowth for long-term fixation and to provide for short term fixation with screw attachments to adjacent vertebral bodies.
However, it is understood that since device 10 is placed in distracted disk space wherein surrounding soft tissue constraints are in tension, that initial screw fixation may not be necessary. Furthermore, bone ingrowth has been shown to occur into ceramic. It is anticipated, therefore, that the entire disk arthroplasty device may be able to be made of only first and second members, 24 and 44, namely the two articulating pieces of ceramic.

An appropriate surgical technique for implacement of the present invention is described in a paper, incorporated herein by reference, entitled "Femoral Cortical Ring Plus Cancellous Dowel: An Alternative in an Anterior Lumbar Interbody Fusion" available from Richard M. Salib, M.D., Institute for Low Back Care, 2800 Chicago Avenue South, Minneapolis, Minnesota 55407.

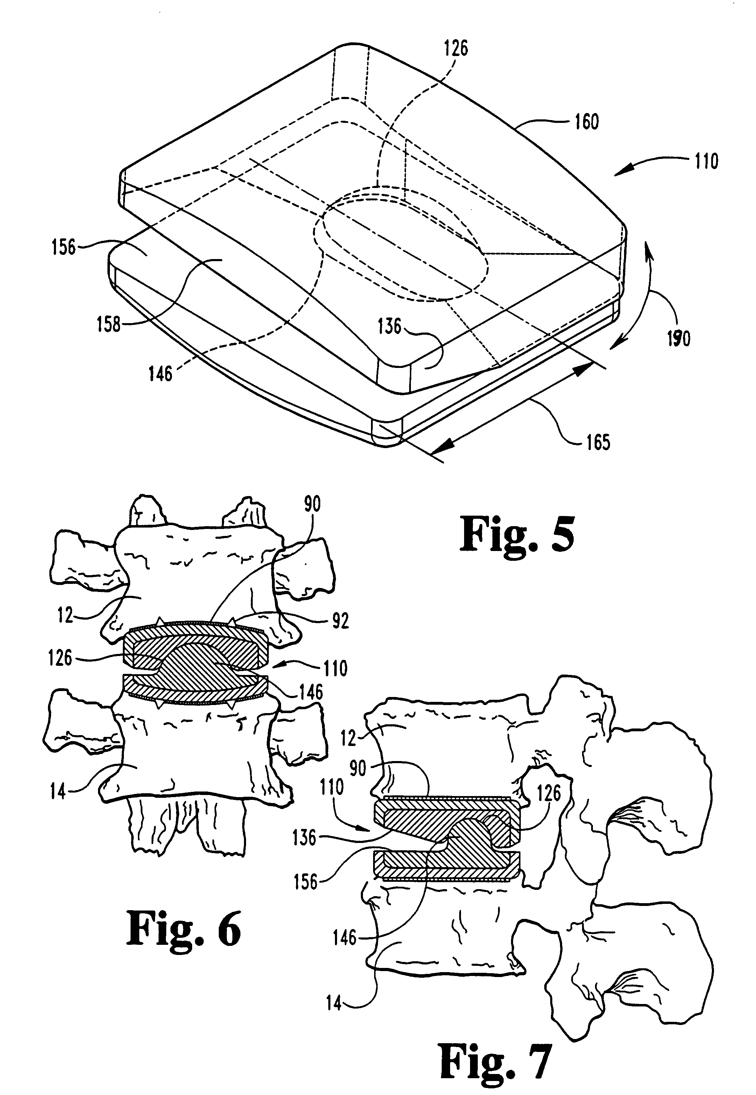

FIGS. 5 - 14 show embodiments of a disk arthroplasty device according to the present invention. Referring to FIG. 5, a disk arthroplasty device 110, similar to that shown in FIGS. 1 - 4, is shown. The disk arthroplasty device 110 comprises an ellipsoidally-shaped ball 146 and socket 126, oriented so that their greatest lengths are disposed along a first axis transverse to the anterior and posterior ends 158 and 160 respectively and their shortest lengths are disposed along a second axis which is perpendicular to the first axis along surface 156. In one preferred embodiment, the radius of the ellipsoidal ball and socket configurations is 0.818 cm (0.322 inches) along the first axis and 0.439 cm (0.173 inches) along the second axis. In another embodiment, the radius of the ball and socket configurations is 1.095 cm (0.431 inches) along the first axis and 0.650 cm (0.256 inches) along the second axis. Generally, the greater the ball radii, the greater the resistance to compressive, rotational and axial shear loading.

The center of rotation 165 of the arthroplasty device 110 is located posteriorly, at 65 percent of the length between the anterior end 158 and posterior end 160, to match the normal lumbar spine center of rotation. The present invention further contemplates locating the device 110 center of rotation elsewhere to match centers of rotation in other areas of the spine.

As seen in FIGS. 5 and 7, the first joint surface 136 is sloped away from socket 126 while the second joint surface 156 remains flat, although the present invention contemplates sloping either one or both of joint surfaces 136 and 156. The degree of slope determines the amount of relative rotation between joint surfaces 136 and 156 respectively and the first joint surface 136 is sloped to provide for up to 5 degrees of lateral bending in either direction, up to 5 degrees of extension and up to 15 degrees of flexion. In the preferred embodiment, the first joint surface 136 is sloped to provide 5 degrees of lateral bending in either direction, 5 degrees of extension and 15 degrees of flexion. In this embodiment, ball 146 and socket 126 may be configured to have a predetermined fit to permit axial rotation of up to 5 degrees in either direction as shown at 190 in FIG. 5. Any further rotation is inhibited due to the elongated nature of ball 146 and socket 126.

FIGS. 6 and 7 show the device arthroplasty device 110 of FIG. 5 positioned between a first and second vertebra 12 and 14 respectively. FIG. 6 shows an anterior view of device arthroplasty disk 110 in place of a natural disk and FIG. 7 shows a side view of the same. Both FIGS. 6 and 7 show the device arthroplasty device 110 of FIG. 5 in cross-section.

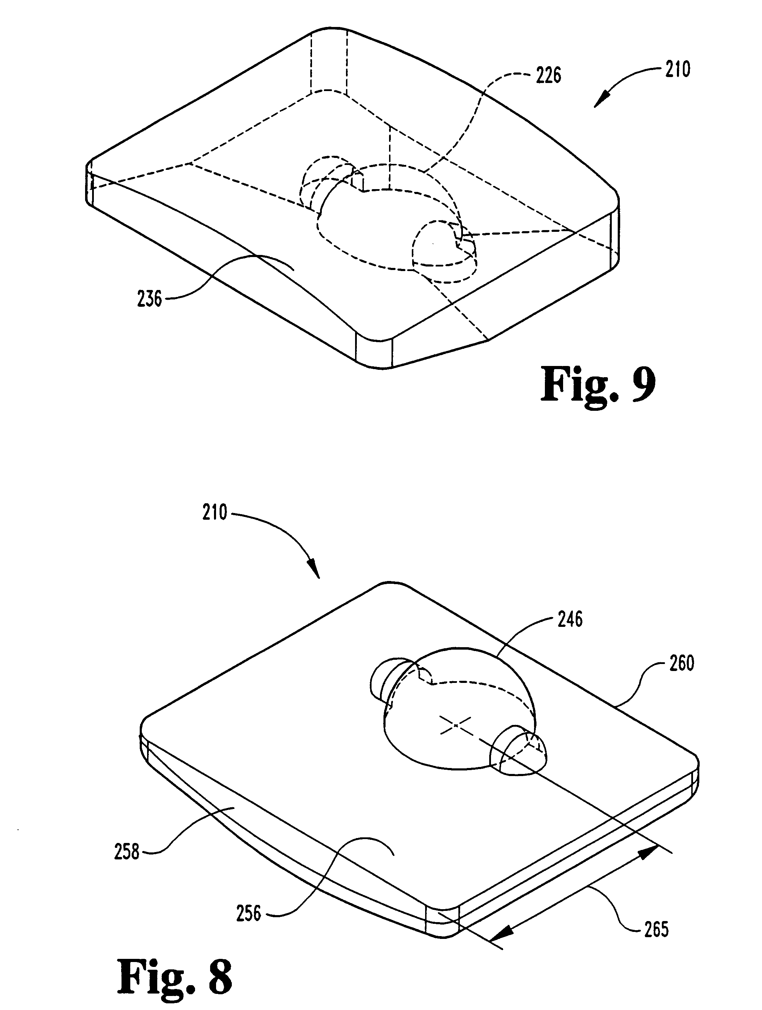

Referring now to FIGS. 8 and 9, another embodiment of the arthroplasty device of the present invention is shown. The device 210 ball 246 of FIG. 8 and socket 226 of FIG. 9 are spherically-shaped with partially cylindrical shapes at the transverse ends.

The center of rotation 265 of the arthroplasty device 210 is located posteriorly, at 65 percent of the length between the anterior end 258 and posterior end 260, to match the normal lumbar spine center of rotation. The present invention further contemplates locating the device 210 center of rotation elsewhere to match centers of rotation in other areas of the spine.

As seen in FIG. 9, the first joint surface 236 is sloped away from socket 226 while the second joint surface 256 remains flat, although the present invention contemplates sloping either one or both of joint surfaces 236 and 256. The degree of slope determines the amount of relative rotation between joint surfaces 236 and 256 respectively and the first joint surface 236 is sloped to provide for up to 5 degrees of lateral bending in either direction, up to 5 degrees of extension and up to 15 degrees of flexion. In the preferred embodiment, the first joint surface 236 is sloped to provide 5 degrees of lateral bending in either direction, 5 degrees of extension and 15 degrees of flexion. In this embodiment, ball 246 and socket 226 may be configured to have a predetermined fit to permit axial rotation of up to 5 degrees in either direction as shown generally at 170 for disk arthroplasty device 110. Any further rotation is inhibited due to the partially cylindrical shapes of ball 246 and socket 226.

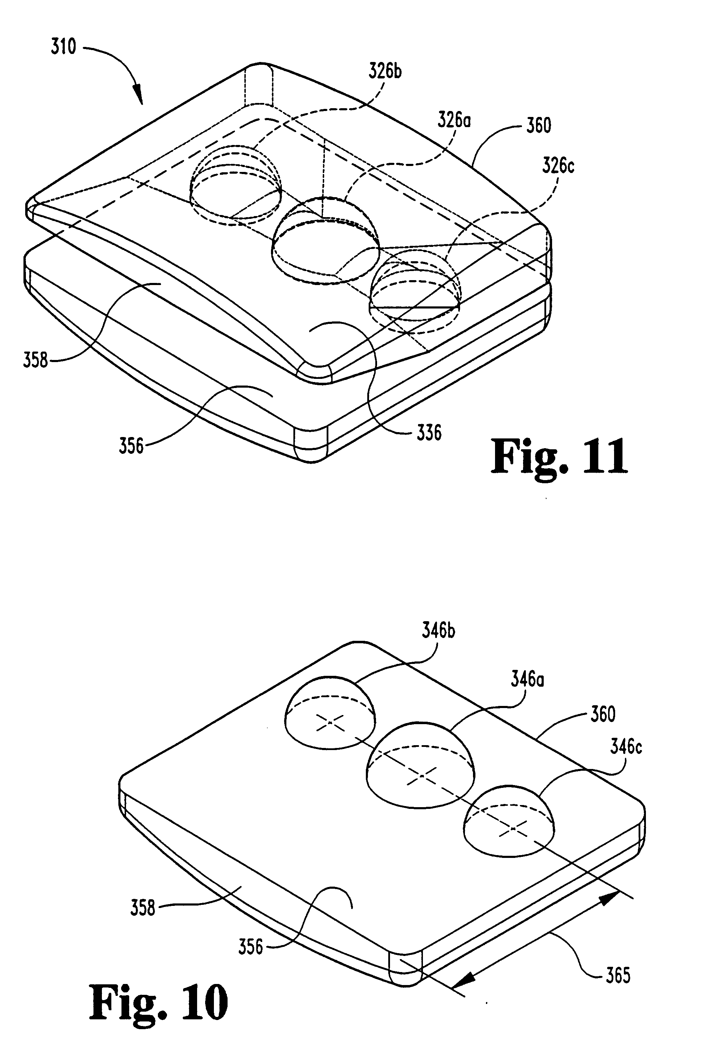

Referring now to FIGS. 10 and 11, another embodiment of arthroplasty device 310 is shown wherein the ball portion 346 of FIG. 10 and corresponding socket portion 326 of FIG. 11 are comprised of three spherical shapes 346(a), 346(b), 346(c), 326(a), 326(b) and 326(c) respectively. Ball 346(a) and socket 326(a) are sized to be larger in radius than the respective (b) and (c) spheres and sockets. In addition, ball and socket portions 346(b), 346(c), 326(b) and 326(c) are sized to have equal radii and be equally spaced in transverse directions from ball and socket portions 346(a) and 326(a) as shown in FIGS. 10 and 11.

The center of rotation 365 of the arthroplasty device 310 is located posteriorly, at 65 percent of the length between the anterior end 358 and posterior end 360, to match the normal lumbar spine center of rotation. The present invention further contemplates locating the device 310 center of rotation elsewhere to match centers of rotation in other areas of the spine.

As seen in FIG. 11, the first joint surface 336 is sloped away from socket 326 while the second joint surface 356 remains flat, although the present invention contemplates sloping either one or both of joint surfaces 336 and 356. The degree of slope determines the amount of relative rotation between joint surfaces 336 and 356 respectively and the first joint surface 336 is sloped to provide for up to 5 degrees of lateral bending in either direction, up to 5 degrees of extension and up to 15 degrees of flexion. In the preferred embodiment, first joint surface 336 is sloped to provide 5 degrees of lateral bending in either direction, 5 degrees of extension and 15 degrees of flexion. In this embodiment, ball portion 346 and socket portion 326 may be configured to have a predetermined fit to permit axial rotation of up to 5 degrees in either direction as shown generally at 170 for disk arthroplasty device 110. Any further rotation is inhibited due to the location of ball 346(b) and corresponding socket 326(b) and ball 346(c) and corresponding socket 326(c).

The design of FIGS. 10 and 11 permit the highest area of contact between ball portion 346 and socket portion 326 while maintaining the required constraint to axial rotation.

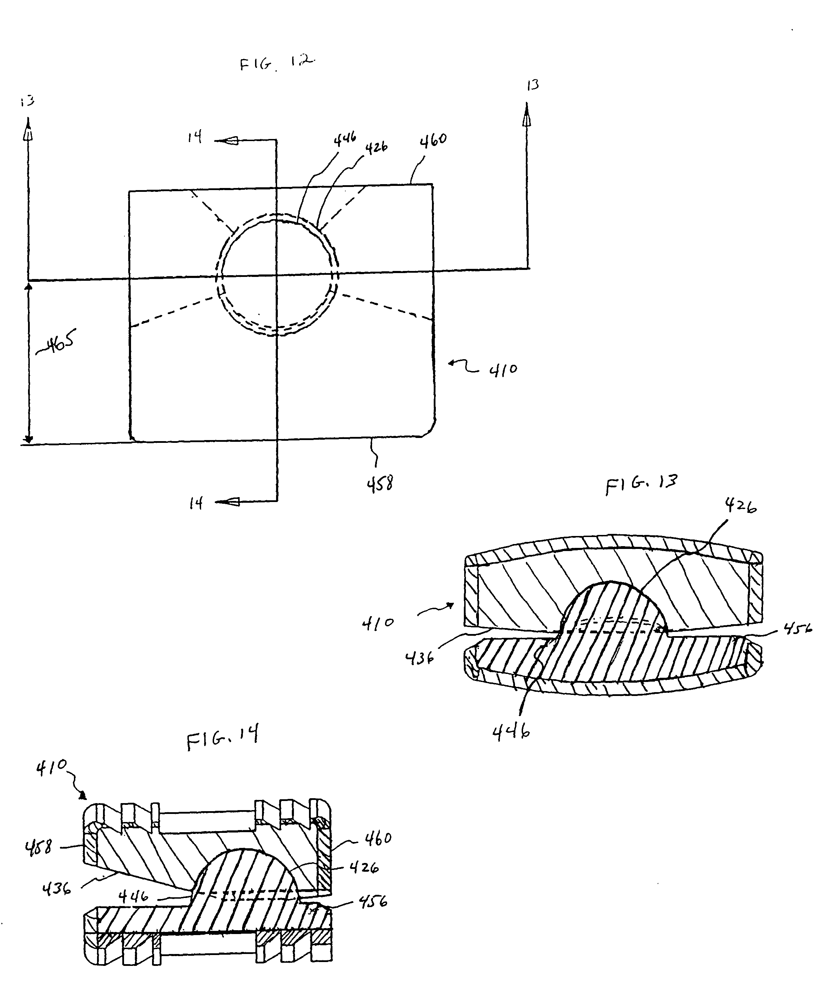

Referring now to FIGS. 12 - 14, another preferred embodiment of arthroplasty device 410 is shown wherein the ball 446 and socket 426 are spherically shaped. This embodiment permits virtually unrestrained rotation about the long axis of the spine. Under normal circumstances, rotation should be restricted to protect the facets. However, if only a small portion of the annulus is removed to insert disk arthroplasty device 410, the remaining annulus is believed to provide significant resistance to rotation, thereby allowing only normal physiological loading of the facets with this design. Restriction of axial rotation is therefore no longer necessary and the disk arthroplasty device may therefore allow 3 degrees of freedom mimicking the normal intervertebral disk movements of flexion/extension, lateral bending and axial rotation. Anterior/posterior shear, lateral shear and axial compression are restricted with this particular embodiment.

The center of rotation 465 of the arthroplasty device 410 is located posteriorly, at 65 percent of the length between the anterior end 458 and posterior end 460, to match the normal lumbar spine center of rotation. The present invention further contemplates locating the device 410 center of rotation elsewhere to match centers of rotation in other areas of the spine.

As seen in FIGS. 13 and 14, the first joint surface 436 is sloped away from socket 426 while the second joint surface 456 remains flat, although the present invention contemplates sloping either one or both of joint surfaces 436 and 456. The degree of slope determines the amount of relative rotation between joint surfaces 436 and 456 respectively and the first joint surface 436 is sloped to provide for up to 5 degrees of lateral bending in either direction, up to 5 degrees of extension and up to 15 degrees of flexion. In the preferred embodiment, the first joint surface is sloped to provide 5 degrees of lateral bending in either direction, 5 degrees of extension and 15 degrees of flexion.

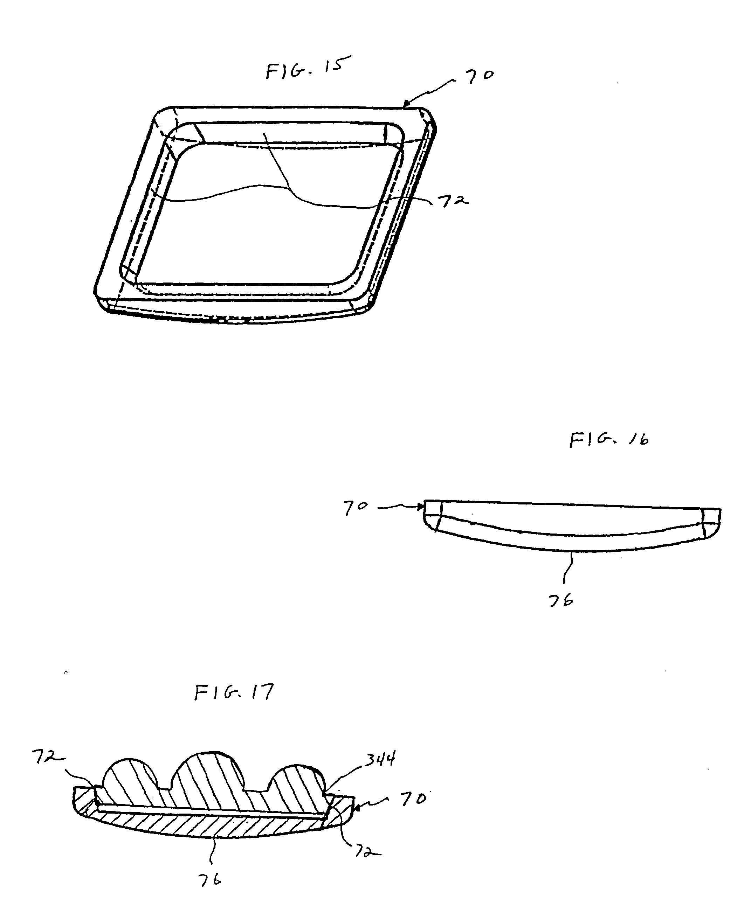

Referring now to FIGS. 15 - 22, each embodiment of the disk arthroplasty device of the present invention may be fastened to insert cups before surgical implantation. Figs. 15 - 17 show one embodiment of an insert cup 70. The insert cup 70 is configured to be fastened to either the upper side of the first member, such as member 324, or the lower side of the second member, such as member 344 as shown in FIG. 17, at its surface 72 by press fitting a disk arthroplasty member within the insert cup. The present invention also contemplates other means for fastening the insert cup 70 to a disk arthroplasty device surface such as with a body-compatible adhesive, tape, solder attachment or clip mechanism. Insert cup 70 is provided with an engaging surface 76 for engaging either vertebral surface as shown in FIGS. 5 and 6. Engaging surface 76 is generally convexly shaped about an axis perpendicular to the spine as shown in FIG. 5. This configuration follows the natural contours of the top and bottom vertebrae surfaces and acts to stabilize the device with respect to axial rotation and anterior/posterior shear.

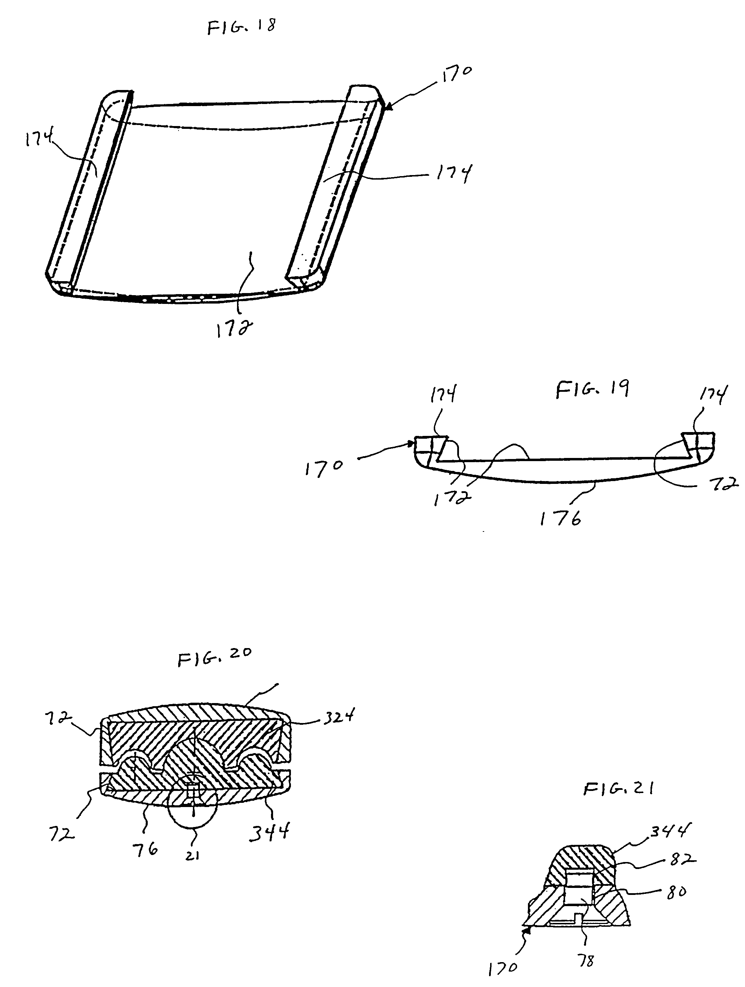

FIGS. 18 - 21 show another embodiment of insert cup 170 with a receiving surface 172 and flanges 174 for slidably receiving the outer surface of a first member, such as member 324, or the outer surface of a second member, such as member 344, as shown in FIG. 20. Insert cup 170 is provided with a convex engaging surface 176 for engaging either vertebral surface, identical to the embodiment shown by insert cup 70. A disk arthroplasty device member is attached to insert cup 170 by screwing the cup 170 to a member, such as member 344, as shown in FIG. 20. As shown in detail in FIG. 21, the screw 78 is counter-sunk through opening 80 in insert cup 170 and engages another opening 82 in member 344.

The preferred material for either insert cup 70 or 170 is a metal, such as titanium. The insert cups may be formed by a variety of known processes such as machining or molding and in one embodiment of the present invention, the insert cups are molded. Using titanium insert cups with a disk arthroplasty device of the present invention provides various benefits such as providing tensile support to the structure and maintaining the first and second member material in compression and providing a structure wherein the disk arthroplasty device members can remain a single size while the insert cup sizes may be more easily and cheaply varied by either adding or reducing metal thickness.

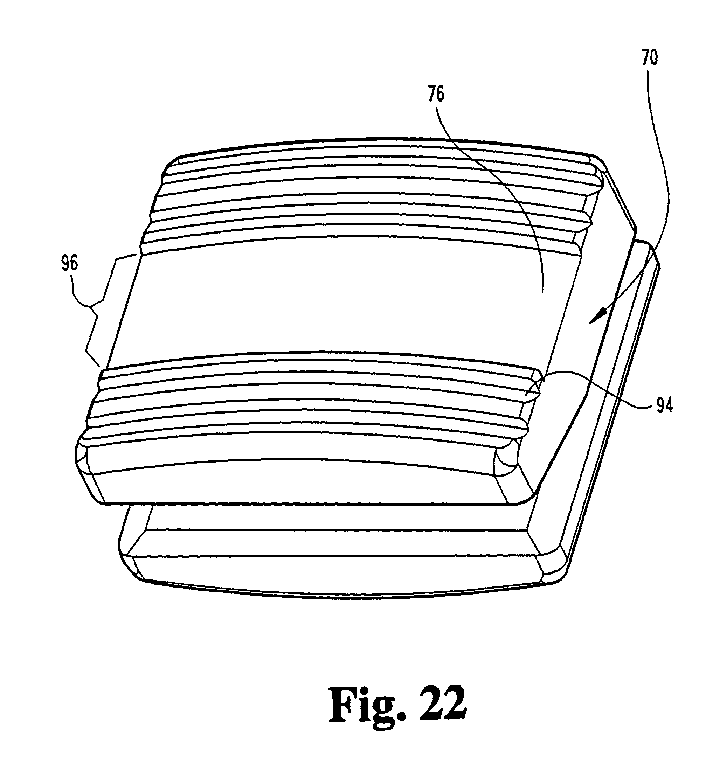

Another benefit of using titanium insert cups with a disk arthroplasty device is that the insert cups provide a surface to which resisting fins, spikes or teeth can be more easily attached than to ceramic. FIG. 6 shows the attachment of resisting fins 92 to the anterior ends of the outer surface of an insert cup. These fins act to enhance the resistance of an arthroplasty device to axial rotation and may be attached to the insert cups by known processes such as machining, soldering, welding and gluing. In one embodiment, the fins are welded to the insert cups. FIG. 22 shows rows of teeth 94 affixed to the surface of insert cup 70 that allow easy insertion of the arthroplasty device into a disk space, but resist expulsion in the opposite direction. Although any of the aforementioned attachment processes may be used to attach the rows of teeth 94 to the insert cups, in one embodiment these teeth are formed into during the machining process of the insert cups.

Yet another benefit of using titanium insert cups with a disk arthroplasty device is that insert cups provide a surface that coatings may easily adhere to. FIGS. 6 and 7 show the application of a titanium bead coating 90 to the outer surfaces of the insert cups to promote bony ingrowth fixation. A variety of known technologies may be used to apply a coating such as sintering or spraying and in one embodiment, the titanium bead coating 90 is sprayed onto the outer surface of the insert cups. FIG. 22 shows a bead pocket 96 that is provided between the sets of teeth rows 94, for receiving a coating of titanium beads. In the embodiment of FIG. 22, the rows of teeth resist expulsion of the disk arthroplasty device opposite to the direction of device insertion. The titanium bead coating disposed within bead pocket 96 then promotes bony ingrowth fixation which acts to hold the outer surface of the insert cups in place and to enhance resistance to movement of the insert cups in any direction.

While the invention has been illustrated and described in detail in the drawings and foregoing description, the same is to be considered as illustrative and not restrictive in character, it being understood that only the preferred embodiment has been shown and described and that all changes and modifications that come within the scope of the invention are desired to be protected.

## Claims

1. An intervertebral disk arthroplasty device (110,210,310,410) adapted to replace a disk between a first vertebra and a second vertebra in a spine, said arthroplasty device comprising:
a first member having a first joint surface (136,236,336,436), a top surface opposite said first joint surface, a first posterior end (160,260,360,460) and a first anterior end (158,258,358,458);
a second member having a second joint surface (156,256,356,456) facing said first joint surface, a bottom surface opposite said second joint surface, and second posterior (160,260,360,460) and anterior ends (158,258,358,458) juxtaposed with corresponding ones of said first posterior and anterior ends;
a first insert cup (70,170) having a first receiving surface (72,172) for receiving said top surface therein, and a first engaging surface (76,176) opposite said first receiving surface for engaging the first vertebra; and
a second insert cup (70,170) having a second receiving surface (72,172) for receiving said bottom surface therein, and a second engaging surface (76,176) opposite said second receiving surface for engaging the second vertebra;
**characterised in that** said first and second engaging surfaces (76,176) are convex about the sagittal plane of the spine;

2. An intervertebral disk arthoplasty device according to claim 1, comprising means (78, 174) for restrainably retaining said top and bottom surfaces within said first and second receiving surfaces (72,172) respectively.

3. The intervertebral disk arthroplasty device of claim 2, wherein said means for restrainably retaining comprises a press fit.

4. The intervertebral disk arthroplasty device of claim 2, wherein said top, bottom, first receiving and second receiving surfaces (72,172) are configured to permit said first and second members to be slidably received within said first and second insert cups (70,170) respectively.

5. The intervertebral disk arthroplasty device of claim 4, wherein said means for restrainably retaining includes:
a first screw (78) oriented in a direction perpendicular to said top surface from said first insert cup (170) toward said first member (324), and engaging said first insert cup (170) to said first member (324); and
a second screw (78) oriented in a direction perpendicular to said bottom surface from said second insert cup (170) toward said second member (344), and engaging said second insert cup (170) to said second member (344).

6. The intervertebral disk arthroplasty device of claim 1 or 2, comprising:
a plurality of resisting means (92) for resisting axial rotation of said intervertebral disk arthroplasty device, said resisting means (92) extending from the anterior ends of said first and second engaging surfaces, and perpendicular to said top and bottom surfaces.

7. The intervertebral disk arthroplasty device of claim 6, comprising:
a coating (90) disposed on said first and second engaging surfaces (76,176) wherein said coating (90) is a biologically acceptable material suitable for promoting bony ingrowth fixation.

8. The intervertebral disk arthroplasty device of claim 7, wherein said material is composed of titanium beads.

9. The intervertebral disk arthroplasty device of claim 1 or 2, comprising:
retaining ribs (94) formed within said top and bottom surfaces and extending parallel with the anterior and posterior ends of said first and second insert cups (70,170) along a substantial portion of said top and bottom surfaces, said retaining ribs (94) being configured to permit movement of said disk arthroplasty device in the first direction and to resist movement of said disk arthroplasty device in an opposite direction.

10. The intervertebral disk arthroplasty device of claim 9, comprising:
a first recessed channel (96) in said top surface, said first channel extending parallel with said retaining ribs (94) along said top surface, said first channel (96) having a first predetermined width centered equidistant from the anterior and posterior ends of said first insert cup (70); and
a second recessed channel (96) in said bottom surface, said second channel (96) extending parallel with said retaining ribs (94) along said bottom surface, said second channel (96) having a second predetermined width centered equidistant from the anterior and posterior ends of said second insert cup (70).

11. The intervertebral disk arthroplasty device of claim 10, comprising:
a coating (90) disposed within said first and second recessed channels (96), wherein said coating (90) is a biologically acceptable material suitable for promoting bony ingrowth fixation.

12. The intervertebral disk arthroplasty device of any preceding claim, wherein said first and second insert cups (70,170) are made of titanium.

13. The intervertebral disk arthroplasty device of any preceding claim, wherein said first and second members are made of zirconia.

14. The intervertebral disk arthroplasty device of any of claims 1 to 12, wherein said first and second members are made of alumina.

15. The intervertebral disk arthroplasty device of claim 1 or 2, wherein said posterior (160,260,360,460) and anterior (158,258,358,458) ends of said first member define a transverse midline therebetween, said midline being equidistant from said first posterior end and said first anterior end;
a ball and socket joint (126,146,226,246,326,346,426,446) is provided between said first and second members defined in said first and second joint surfaces (136,156,236,256,336,356,436,456) and disposed between said midline and said first posterior end, said ball and socket joint being configured to permit relative rotation between said first member and said second member about a first axis parallel to said transverse midline and a second axis perpendicular to said first axis and lying in a sagittal plane of the spine, wherein one of said first and second joint surfaces (136,156,236,256,336,356,436,456) is inclined away from said ball and socket joint (126,146,226,246,326,346,426,446) toward its respective first or second joint member, entirely around said joint, and the other of said first and second joint surfaces is substantially parallel to a plane defined by said first and second axes, around said joint.

16. The intervertebral disk arthroplasty device of claim 15, wherein said inclined surface includes a first inclined face extending from said first axis to said anterior end at a first angle, a second inclined face extending from said first axis to said posterior end at a second angle, third and fourth inclined faces extending from said second axis in opposite directions away from said second axis and parallel to said first axis at third and fourth angles respectively, said first, second, third and fourth angles being measured relative to the other of said first and second joint surfaces (136,156,236,256,336,356,436,456).

17. The intervertebral disk arthroplasty device of claim 16, wherein said third and fourth angles are sized to permit relative rotation up to a first predetermined angle between said first and second members about said second axis before said first joint surface (136,236,336,436) contacts said second joint surface (156,256,356,456).

18. The intervertebral disk arthroplasty device of claim 17, wherein said second angle is sized to permit relative rotation between said first and second members, up to a second predetermined angle in a direction toward said posterior ends (160,260,360,460), about said first axis before said first joint surface (136,236,336,436) contacts said second joint surface (156,256,356,456).

19. The intervertebral disk arthroplasty device of claim 18, wherein said first angle is sized to permit relative rotation between said first and second members, up to a third predetermined angle in a direction toward said anterior ends, about said first axis before said first joint surface (136,236,336,436) contacts said second joint surface (156,256,356,456).

20. The intervertebral disk arthroplasty device of claim 19, wherein said ball and socket joint (126,146,226,246,326,346,426,446) includes a ball portion (146,246,346,446) defined on said first joint surface (136,236,336,436) of said first member along said first axis and a socket portion (126,226,326,426) correspondingly defined in said second joint surface (156,256,356,456) of said second member to receive said ball portion therein.

21. The intervertebral disk arthroplasty device of claim 20, wherein said ball portion (146,246,346,446) is circular in cross section along said first joint surface.

22. The intervertebral disk arthroplasty device of claim 15, wherein said ball and socket joint (126,146) is elongated and comprises a ball portion (146) defined on said first joint surface (136) of said first member and elongated along a first axis parallel to said transverse midline, and
a socket portion (126) correspondingly defined in said second joint surface (156) of said second member for receiving said elongated ball portion (146) therein with a predetermined fit to permit a predetermined relative rotation between said first and second members about a second axis perpendicular to said first and second joint surfaces (136,156) before said elongated ball portion (146) contacts said corresponding socket portion (126).

23. The intervertebral disk arthroplasty device of claim 22, wherein said elongated ball and socket portions (146,126) are ellipsoidal in cross section in a plane parallel to said first joint surface.

24. The intervertebral disk arthroplasty device of claim 22, wherein said elongated ball and socket portions (226,246) are partially circular in cross section in a plane parallel to said first joint surface and include opposite ends formed in a partially cylindrical shape.

25. The intervertebral disk arthroplasty device of claim 22, wherein said elongated ball and socket joint (326,346) includes a plurality of separate ball portions and corresponding socket portions.

26. The intervertebral disk arthroplasty device of claim 25, wherein said plurality of ball and socket portions (326,346) include:
a first ball and socket portion (326a,346a); and
second (326b,346b) and third (326c,346c) ball and socket portions spaced on opposite sides of said first ball portion (346a) and equidistant from said first ball portion.

27. The intervertebral disk arthroplasty device of claim 26, wherein said first (346a), second (346b) and third (346c) ball portions are circular in cross section in a plane parallel to said first joint surface, said first ball portion (346a) having a radius greater than the radii of said second (346b) and third (346c) ball portions.

28. The intervertebral disk arthroplasty device of claim 27, wherein said second (346b) and third ball (346c) portions have equal radii.

29. The intervertebral disk arthroplasty device of claim 1 or 2, wherein posterior (360) and anterior (358) ends of said first member define a transverse midline therebetween, said midline being equidistant from said first posterior end and said first anterior end;
a joint is provided between said first and second members to permit relative rotation between said first member and said second member about a first axis parallel to said transverse midline and a second axis perpendicular to said first axis, said joint including;
a first (346a), second (346b) and third (346c) ball portion defined in said first joint surface (336) of said first member, said ball portions having circular cross sections along an axis parallel to said transverse midline, said first ball portion (346a) having a larger radius than said second (346b) and third (346c) ball portions, said second and third ball portions having equal radii and being disposed on opposite sides of said first ball portion, said ball portions being disposed radially about said first axis; and
a socket portion (326a,326b,326c) defined in said second joint surface substantially complementary to said ball portions (346a,346b,346c) in said first joint surface (336) and configured to receive said ball portions therein;
said engaging surfaces of said first and second insert cups (70,170) being disposed convexly about said second axis.

## Patentansprüche

1. Zwischenwirbelscheiben-Arthroplastievorrichtung (110, 210, 310, 410), die dafür geeignet ist, eine Scheibe zwischen einem ersten Wirbel und einem zweiten Wirbel in einer Wirbelsäule zu ersetzen, wobei die Arthroplastievorrichtung folgendes aufweist:
ein erstes Element mit einer ersten Verbindungsfläche (136, 236, 336, 436), einer oberen Fläche gegenüber der ersten Verbindungsfläche, einem ersten hinteren Ende (160, 260, 360, 460) und einem ersten vorderen Ende (158, 258, 358, 458),
ein zweites Element mit einer zweiten Verbindungsfläche (156, 256, 356, 456), die zu der ersten Verbindungsfläche hin zeigt, einer unteren Fläche gegenüber der zweiten Verbindungsfläche, und zweite hintere (160, 260, 360, 460) und vordere (158, 258, 358, 458) Ende, die in Juxtaposition mit entsprechenden der ersten hinteren und vorderen Enden angeordnet sind,
eine erste Einsatzschale (70, 170) mit einer ersten Aufnahmefläche (72, 172), um darin die obere Fläche aufzunehmen, und eine erste Eingriffsfläche (76, 176) gegenüber der ersten Aufnahmefläche zum Ineinandergreifen mit dem ersten Wirbel, und
eine zweite Einsatzschale (70, 170) mit einer zweiten Aufnahmefläche (72, 172), um darin die untere Fläche aufzunehmen, und eine zweite Eingriffsfläche (76, 176) gegenüber der zweiten Aufnahmefläche zum Ineinandergreifen mit dem zweiten Wirbel,
**dadurch gekennzeichnet, daß** die erste und zweite Eingriffsfläche (76, 176) konvex um die Sagittalebene der Wirbelsäule sind.

2. Zwischenwirbelscheiben-Arthroplastievorrichtung nach Anspruch 1, die Mittel (78, 174) um die obere und untere Fläche beschränkend innerhalb der ersten bzw. zweiten Aufnahmefläche (72, 172) zu halten, aufweist.

3. Zwischenwirbelscheiben-Arthroplastievorrichtung nach Anspruch 2, bei der die Mittel zum beschränkenden Halten eine Preßpassung aufweisen.

4. Zwischenwirbelscheiben-Arthroplastievorrichtung nach Anspruch 2, bei der die obere, untere erste Aufnahme- und zweite Aufnahmefläche (72, 172) so konfiguriert sind, daß die sie gleitende Aufnahme des ersten und zweiten Elements innerhalb der ersten bzw. zweiten Einsatzschale (70, 172) erlauben.

5. Zwischenwirbelscheiben-Arthroplastievorrichtung nach Anspruch 4, bei der die Mittel zum beschränkenden Halten folgendes einschließen:
eine erste Schraube (78), die in einer Richtung senkrecht zu der oberen Fläche von der ersten Einsatzschale (170) hin zu dem ersten Element (324) ausgerichtet ist und die erste Einsatzschale (170) mit dem ersten Element (324) zum Eingriff bringt, und
eine zweite Schraube (78), die in einer Richtung senkrecht zu der unteren Fläche von der zweiten Einsatzschale (170) hin zu dem zweiten Element (344) ausgerichtet ist und die zweite Einsatzschale (170) mit dem zweiten Element (344) zum Eingriff bringt.

6. Zwischenwirbelscheiben-Arthroplastievorrichtung nach Anspruch 1 oder 2, die folgendes aufweist:
eine Vielzahl von Widerstandsmitteln (92), um der Axialdrehung der Zwischenwirbelscheiben-Arthroplastievorrichtung entgegenzuwirken, wobei die Widerstandsmittel (92) von den vorderen Enden der ersten und zweiten Eingriffsflächen ausgehen und senkrecht zu den oberen und unteren Flächen verlaufen.

7. Zwischenwirbelscheiben-Arthroplastievorrichtung nach Anspruch 6, die folgendes aufweist:
einen Überzug (90), der auf der ersten und zweiten Eingriffsfläche (76, 176) angeordnet ist, wobei der Überzug (90) ein biologisch akzeptierbares Material ist, das die Fixierung durch Knocheneinwuchs fördert.

8. Zwischenwirbelscheiben-Arthroplastievorrichtung nach Anspruch 7, bei der das Material aus Titankügelchen besteht.

9. Zwischenwirbelscheiben-Arthroplastievorrichtung nach Anspruch 1 oder 2, die folgendes aufweist:
Halterippen (94), die innerhalb der oberen und unteren Fläche gebildet werden und parallel mit den vorderen und hinteren Enden der ersten und zweiten Einsatzschalen (70, 170) längs eines beachtlichen Abschnitts der oberen und unteren Flächen verlaufen, wobei die Halterippen (94) dafür konfiguriert sind, die Bewegung der Scheiben-Arthroplastievorrichtung in der ersten Richtung zu erlauben und der Bewegung der Scheiben-Arthroplastievorrichtung in einer entgegengesetzten Richtung entgegenzuwirken.

10. Zwischenwirbelscheiben-Arthroplastievorrichtung nach Anspruch 9, die folgendes aufweist:
eine erste ausgesparte Rille (96) in der oberen Fläche, wobei die erste Rille parallel mit den Halterippen (94) längs der oberen Fläche verläuft, wobei die erste Rille (96) eine erste vorher festgelegte Breite hat, die im gleichen Abstand von den vorderen und hinteren Enden der ersten Einsatzschale (70) zentriert ist, und
eine zweite ausgesparte Rille (96) in der unteren Fläche, wobei die zweite Rille parallel mit den Halterippen (94) längs der unteren Fläche verläuft, wobei die zweite Rille (96) eine zweite vorher festgelegte Breite hat, die im gleichen Abstand von den vorderen und hinteren Enden der zweiten Einsatzschale (70) zentriert ist.

11. Zwischenwirbelscheiben-Arthroplastievorrichtung nach Anspruch 10, die folgendes aufweist:
einen Überzug (90), der innerhalb der ersten und zweiten ausgesparten Rillen (96) angeordnet ist, wobei der Überzug (90) ein biologisch akzeptierbares Material ist, das die Fixierung durch Knocheneinwuchs fördert.

12. Zwischenwirbelscheiben-Arthroplastievorrichtung nach einem der vorhergehenden Ansprüche, bei der die erste und zweite Einsatzschale (70, 170) aus Titan hergestellt werden.

13. Zwischenwirbelscheiben-Arthroplastievorrichtung nach einem der vorhergehenden Ansprüche, bei der das erste und zweite Element aus Zircondioxid hergestellt werden.

14. Zwischenwirbelscheiben-Arthroplastievorrichtung nach einem der Ansprüche 1 bis 12, bei der das erste und zweite Element aus Aluminiumoxid hergestellt werden.

15. Zwischenwirbelscheiben-Arthroplastievorrichtung nach Anspruch 1 oder 2, bei der die hinteren (160, 260, 360, 460) und vorderen (158, 258, 358, 458) Enden des ersten Elements zwischen sich eine querverlaufende Mittellinie definieren, wobei die Mittellinie im gleichen Abstand zu dem ersten hinteren und dem ersten vorderen Ende angeordnet ist,
ein Kugelpfannengelenk (126, 146, 226, 246, 326, 346, 426, 446) zwischen den ersten und zweiten Elementen bereitgestellt wird, die in den ersten und zweiten Verbindungsflächen (136, 156, 236, 256, 336, 356, 436, 456) definiert werden und zwischen der Mittellinie und dem ersten hinteren Ende angeordnet sind, wobei das Kugelpfannengelenk so konfiguriert ist, daß es die relative Drehung zwischen dem ersten Element und dem zweiten Elernent um eine erste Achse parallel zu der querverlaufenden Mittellinie und eine zweite Achse senkrecht zu der ersten Achse und in einer Sagittalebene der Wirbelsäule liegend zuläßt, bei der eine der ersten und zweiten Verbindungsflächen (136, 156, 236, 256, 336, 356, 436, 456) von dem Kugelpfannengelenk (126, 146, 226, 246, 326, 346, 426, 446) weg zu dem entsprechenden ersten oder zweiten Verbindungselement hin, vollständig um dieses Gelenk, geneigt ist und die andere der ersten und zweiten Verbindungsflächen im Wesentlichen parallel zu einer Ebene ist, die durch die ersten und zweiten Achsen definiert wird, um das Gelenk.

16. Zwischenwirbelscheiben-Arthroplastievorrichtung nach Anspruch 15, bei der die geneigte Oberfläche folgendes einschließt: eine erste geneigte Fläche, die sich in einem ersten Winkel von der ersten Achse zu dem vorderen Ende erstreckt, eine zweite geneigte Fläche, die sich in einem zweiten Winkel von der ersten Achse zu dem hinteren Ende erstreckt, dritte und vierte geneigte Flächen, die sich in dritten bzw. vierten Winkeln von der zweiten Achse in entgegengesetzten Richtungen weg von der zweiten Achse und parallel zu der ersten Achse erstrecken, wobei der erste, zweite, dritte und vierte Winkel im Verhältnis zu der anderen der ersten und zweiten Verbindungsflächen (136, 156, 236, 256, 336, 356, 436, 456) gemessen werden.

17. Zwischenwirbelscheiben-Arthroplastievorrichtung nach Anspruch 16, bei welcher der dritte und vierte Winkel so bemessen sind, daß sie die relative Drehung bis zu einem vorher festgelegten Winkel um die zweite Achse zwischen dem ersten und zweiten Element ermöglichen, bevor die erste Verbindungsfläche (136, 236, 336, 436) die zweite Verbindungsfläche (156, 256, 356, 456) kontaktiert.

18. Zwischenwirbelscheiben-Arthroplastievorrichtung nach Anspruch 17, bei welcher der zweite Winkel so bemessen ist, daß er bis zu einem zweiten vorher festgelegten Winkel die relative Drehung zwischen dem ersten und zweiten Element in einer Richtung hin zu den hinteren Enden (160, 260, 360, 460) um die erste Achse ermöglicht, bevor die erste Verbindungsfläche (136, 236, 336, 436) die zweite Verbindungsfläche (156, 256, 356, 456) kontaktiert.

19. Zwischenwirbelscheiben-Arthroplastievorrichtung nach Anspruch 18, bei welcher der erste Winkel so bemessen ist, daß er bis zu einem dritten vorher festgelegten Winkel die relative Drehung zwischen dem ersten und zweiten Element in einer Richtung hin zu den vorderen Enden um die erste Achse ermöglicht, bevor die erste Verbindungsfläche (136, 236, 336, 436) die zweite Verbindungsfläche (156, 256, 356, 456) kontaktiert.

20. Zwischenwirbelscheiben-Arthroplastievorrichtung nach Anspruch 19, bei der das Kugelpfannengelenk (126, 146, 226, 246, 326, 346, 426, 446) einen Kugelabschnitt (146, 246, 346, 446), der auf der ersten Verbindungsfläche (136, 236, 336, 436) des ersten Elements längs der ersten Achse definiert wird und einen Pfannenabschnitt (126, 226, 326, 426) einschließt, der entsprechend in der zweiten Verbindungsfläche (156, 256, 356, 456) des zweiten Elements definiert wird, um darin den Kugelabschnitt aufzunehmen.

21. Zwischenwirbelscheiben-Arthroplastievorrichtung nach Anspruch 20, bei welcher der Kugelabschnitt (146, 246, 346, 446) längs der ersten Verbindungsfläche einen runden Querschnitt hat.

22. Zwischenwirbelscheiben-Arthroplastievorrichtung nach Anspruch 15, bei der das Kugelpfannengelenk (126, 146) länglich ist und einen Kugelabschnitt (146) aufweist, der auf der ersten Verbindungsfläche (136) des ersten Elements definiert wird und längs einer ersten Achse parallel zu der quer verlaufenden Mittellinie gestreckt ist, und
einen Pfannenabschnitt (126), der entsprechend in der zweiten Verbindungsfläche (156) des zweiten Elements definiert wird, um darin den länglichen Kugelabschnitt (146) mit einer vorher festgelegten Passung aufzunehmen, um eine vorher festgelegte relative Drehung zwischen dem ersten und zweiten Element um eine zweite Achse senkrecht zu der ersten und zweiten Verbindungsfläche (136, 156) zu ermöglichen, bevor der längliche Kugelabschnitt (146) den entsprechenden Pfannenabschnitt (126) kontaktiert.

23. Zwischenwirbelscheiben-Arthroplastievorrichtung nach Anspruch 22, bei der die länglichen Kugel- und Pfannenabschnitte (146, 126) in einer Ebene parallel zu der ersten Verbindungsfläche einen ellipsoiden Querschnitt haben.

24. Zwischenwirbelscheiben-Arthroplastievorrichtung nach Anspruch 22, bei der die länglichen Kugel- und Pfannenabschnitte (226, 246) in einer Ebene parallel zu der ersten Verbindungsfläche einen runden Querschnitt haben und gegenüberliegende Enden einschließen, die in einer partiell zylindrischen Form ausgeführt sind.

25. Zwischenwirbelscheiben-Arthroplastievorrichtung nach Anspruch 22, bei der die länglichen Kugel- und Pfannenabschnitte (326, 346) eine Vielzahl von getrennten Kugelabschnitten und entsprechenden Pfannenabschnitten einschließt.

26. Zwischenwirbelscheiben-Arthroplastievorrichtung nach Anspruch 25, bei der die Vielzahl von Kugel- und Pfannenabschnitten (326, 346) folgendes einschließt:
einen ersten Kugel- und Pfannenabschnitt (326a, 346a) und
zweite (326b, 346b) und dritte (326c, 346c) Kugel- und Pfannenabschnitte, die mit Zwischenraum auf gegenüberliegenden Seiten des ersten Kugelabschnitts (346a) und im gleichen Abstand von dem ersten Kugelabschnitt angeordnet sind.

27. Zwischenwirbelscheiben-Arthroplastievorrichtung nach Anspruch 26, bei der die ersten (346a), zweiten (346b) und dritten (346c) Kugelabschnitte in einer Ebene parallel zu der ersten Verbindungsfläche einen runden Querschnitt haben, wobei der erste Kugelabschnitt (346a) einen größeren Radius als die Radien der zweiten (346b) und dritten (346c) Kugelabschnitte hat.

28. Zwischenwirbelscheiben-Arthroplastievorrichtung nach Anspruch 27, bei der die zweiten (346b) und dritten (346c) Kugelabschnitte gleiche Radien haben.

29. Zwischenwirbelscheiben-Arthroplastievorrichtung nach Anspruch 1 oder 2, bei der das hintere (360) und das vordere (358) Ende des ersten Elements zwischen sich eine querverlaufende Mittellinie definiert, wobei die Mittellinie einen gleichen Abstand von dem ersten hinteren Ende und dem ersten vorderen Ende hat,
zwischen dem ersten und zweiten Element ein Gelenk bereitgestellt wird, um eine relative Drehung zwischen dem ersten Element und dem zweiten Element um eine erste Achse parallel zu der querverlaufenden Mittellinie und eine zweite Achse senkrecht zu der ersten Achse zu ermöglichen, wobei das Gelenk folgendes einschließt:
einen ersten (346a), zweiten (346b) und dritten (346c) Kugelabschnitt, die in der ersten Verbindungsfläche (336) des ersten Elements definiert werden, wobei die Kugelabschnitte längs einer Achse parallel zu der querverlaufenden Mittellinie runde Querschnitte haben, wobei der erste Kugelabschnitt (346a) einen größeren Radius als der zweite (346b) und dritte (346c) Kugelabschnitt hat, wobei der zweite und dritte Kugelabschnitt gleiche Radien haben und auf gegenüberliegenden Seiten des ersten Kugelabschnitts angeordnet sind, wobei die Kugelabschnitte in Radialrichtung um die erste Achse angeordnet sind, und
einen Pfannenabschnitt (326a, 326b, 326c), der in der zweiten Verbindungsfläche im wesentlichen komplementär zu den Kugelabschnitten (346a, 346b, 346c) in der ersten Verbindungsfläche (336) definiert wird und für die Aufnahme der Kugelabschnitte definiert ist,
wobei die Eingriffsflächen der ersten und zweiten Einsatzschale (70, 170) konvex um die zweite Achse angeordnet sind.

## Revendications

1. Dispositif d'arthroplastie de disque intervertébral (110, 210, 310, 410) destiné à remplacer un disque entre une première vertèbre et une deuxième vertèbre dans une colonne vertébrale, ledit dispositif d'arthroplastie comprenant:
un premier élément comportant une première surface d'articulation (136, 236, 336, 436), une surface supérieure opposée à ladite première surface d'articulation, une première extrémité postérieure (160, 260, 360, 460) et une première extrémité antérieure (158, 258, 358, 458);
un deuxième élément comportant une deuxième surface d'articulation (156, 256, 356, 456) faisant face à ladite première surface d'articulation, une surface inférieure opposée à ladite deuxième surface d'articulation et des deuxièmes extrémités postérieure (160, 260, 360, 460) et antérieure (158, 258, 358, 458), juxtaposées aux premières extrémités postérieure et antérieure correspondantes;
une première coupelle d'insertion (70,170), comportant une première surface de réception (72, 172) pour recevoir ladite surface supérieure, et une première surface d'engagement (76, 176) opposée à ladite première surface de réception en vue d'un engagement dans la première vertèbre; et
une deuxième coupelle d'insertion (70, 170) comportant une deuxième surface de réception (72, 172) pour recevoir ladite surface inférieure, et une deuxième surface d'engagement (76, 176) opposée à ladite deuxième surface de réception en vue de l'engagement dans la deuxième vertèbre;
**caractérisé en ce que** lesdites première et deuxième surfaces d'engagement (76, 176) sont convexes autour du plan sagittal de la colonne vertébrale.

2. Dispositif d'arthroplastie de disque intervertébral selon la revendication 1, comprenant un moyen (78, 174) pour retenir et restreindre respectivement lesdites surfaces supérieure et inférieure dans lesdites première et deuxième surfaces de réception (72, 172).

3. Dispositif d'arthroplastie de disque intervertébral selon la revendication 2, dans lequel ledit moyen destiné à une retenue à restriction comprend un ajustement serré.

4. Dispositif d'arthroplastie de disque intervertébral selon la revendication 2, dans lequel lesdites surfaces supérieure, inférieure ainsi que les première et deuxième surfaces de réception (72, 172) sont configurées de sorte à permettre respectivement la réception par glissement desdits premier et deuxième éléments dans lesdites première et deuxième coupelles d'insertion (70, 170).

5. Dispositif d'arthroplastie de disque intervertébral selon la revendication 4, dans lequel ledit moyen destiné à une retenue à restriction englobe:
une première vis (78) orientée dans une direction perpendiculaire à ladite surface supérieure à partir de ladite première coupelle d'insertion (170), en direction dudit premier élément (324), et entraînant l'engagement de ladite première coupelle d'insertion (170) dans ledit premier élément (324); et
une deuxième vis (78), orientée dans une direction perpendiculaire à ladite surface inférieure à partir de ladite deuxième coupelle d'insertion (170), en direction dudit deuxième élément (344), et entraînant l'engagement de ladite deuxième coupelle d'insertion (170) dans ledit deuxième élément (344).

6. Dispositif d'arthroplastie de disque intervertébral selon la revendication 1 ou 2, comprenant:
plusieurs moyens de résistance (92) destinés à résister à la rotation axiale dudit dispositif d'arthroplastie de disque intervertébral, lesdits moyens de résistance (92) s'étendant à partir des extrémités antérieures desdites première et deuxième surfaces d'engagement, de manière perpendiculaire auxdites surfaces supérieure et inférieure.

7. Dispositif d'arthroplastie de disque intervertébral selon la revendication 6, comprenant:
un revêtement (90) agencé sur lesdites première et deuxième surfaces d'engagement (76, 176), ledit revêtement (90) étant composé d'un matériau biologiquement acceptable appropriée à faciliter la fixation par croissance osseuse.

8. Dispositif d'arthroplastie de disque intervertébral selon la revendication 7, dans lequel ledit matériau est composé de billes de titane.

9. Dispositif d'arthroplastie de disque intervertébral selon la revendication 1 ou 2, comprenant:
des nervures de retenue (94) formées dans lesdites surfaces supérieure et inférieure et s'étendant parallèlement aux extrémités antérieure et postérieure desdites première et deuxième coupelles d'insertion (70, 170), le long d'une partie substantielle desdites surfaces supérieure et inférieure, lesdites nervures de retenue (94) étant configurées de sorte à permettre le déplacement dudit dispositif d'arthroplastie de disque intervertébral dans la première direction et à résister au déplacement dudit dispositif d'arthroplastie de disque intervertébral dans une direction opposée.

10. Dispositif d'arthroplastie de disque intervertébral selon la revendication 9, comprenant:
un premier canal évidé (96) dans ladite surface supérieure, ledit premier canal s'étendant parallèlement auxdites nervures de retenue (94) le long de ladite surface supérieure, ledit premier canal (96) ayant une première largeur prédéterminée centrée à une distance égale des extrémités antérieure et postérieure de ladite première coupelle d'insertion (70); et
un deuxième canal évidé (96) dans ladite surface inférieure, ledit deuxième canal (96) s'étendant parallèlement auxdites nervures de retenue (94) le long de ladite surface inférieure, ledit deuxième canal (96) ayant une deuxième largeur prédéterminée centrée à une distance égale des extrémités antérieure et postérieure de ladite deuxième coupelle d'insertion (70).

11. Dispositif d'arthroplastie de disque intervertébral selon la revendication 10, comprenant:
un revêtement (90) agencé dans lesdits premier et deuxième canaux évidés (96), ledit revêtement (90) étant composé d'un matériau biologiquement acceptable pour faciliter la fixation par croissance osseuse.

12. Dispositif d'arthroplastie de disque intervertébral selon l'une quelconque des revendications précédentes, dans lequel lesdites première et deuxième coupelles d'insertion (70, 170) sont composées de titane.

13. Dispositif d'arthroplastie de disque intervertébral selon l'une quelconque des revendications précédentes, dans lequel lesdits premier et deuxième éléments sont composés de zircone.

14. Dispositif d'arthroplastie de disque intervertébral selon l'une quelconque des revendications 1 à 12, dans lequel lesdits premier et deuxième éléments sont composés d'aluminium.

15. Dispositif d'arthroplastie de disque intervertébral selon la revendication 1 ou 2, dans lequel lesdites extrémités postérieure (160, 260, 360, 460) et antérieure (158, 258, 358, 458) dudit premier élément définissent une ligne médiane transversale entre elles, ladite ligne médiane étant située à une distance égale de ladite première extrémité postérieure et de ladite première extrémité antérieure;
une articulation à rotule et logement de rotule (126, 146, 226, 246, 326, 346, 426, 446) étant agencée entre lesdits premier et deuxièmes éléments définis dans lesdites première et deuxième surfaces d'articulation (136, 156, 236, 256, 336, 356, 436, 456) et agencées entre ladite ligne médiane et ladite première extrémité postérieure, ladite articulation à rotule et logement de rotule étant configurée de sorte à permettre la rotation relative entre ledit premier élément et ledit deuxième élément autour d'un premier axe parallèle à ladite ligne médiane transversale et un deuxième axe perpendiculaire audit premier axe et situé dans un plan sagittal de la colonne vertébrale, une desdites première et deuxième surfaces d'articulation (136, 156, 236, 256, 336, 356, 436, 456) étant inclinée à l'écart de ladite articulation à rotule et logement de rotule (126, 146, 226, 246, 326, 346, 426, 446) en direction de son premier ou deuxième élément d'articulation respectif, entourant entièrement ladite articulation, l'autre desdites première et deuxième surfaces d'articulation étant pratiquement parallèle à un plan défini par lesdits premier et deuxième axes, autour de ladite articulation.

16. Dispositif d'arthroplastie de disque intervertébral selon la revendication 15, dans lequel ladite surface inclinée englobe une première face inclinée s'étendant dudit premier axe vers ladite extrémité antérieure à un premier angle, une deuxième face inclinée s'étendant à partir dudit premier axe vers ladite extrémité postérieure à un deuxième angle, des troisième et quatrième faces inclinées s'étendant à partir dudit deuxième axe dans des directions opposées, à l'écart dudit deuxième axe et parallèlement audit premier axe, respectivement à des troisième et quatrième angles, lesdits premier, deuxième troisième et quatrième angles étant mesurés par rapport à l'autre desdites première et deuxième surfaces d'articulation (136, 156, 236, 256, 336, 356, 436, 456).

17. Dispositif d'arthroplastie de disque intervertébral selon la revendication 16, dans lequel lesdits troisième et quatrième angles sont dimensionnés de sorte à permettre la rotation relative jusqu'à un premier angle prédéterminé entre lesdits premier et deuxième éléments autour dudit deuxième axe avant que ladite première surface d'articulation (136, 236, 336, 436) contacte ladite deuxième surface d'articulation (156, 256, 356, 456).

18. Dispositif d'arthroplastie de disque intervertébral selon la revendication 17, dans lequel ledit deuxième angle est dimensionné de sorte à permettre une rotation relative entre lesdites premier et deuxième éléments, jusqu'à un deuxième angle prédéterminé, dans une direction allant vers lesdites extrémités postérieures (160, 260, 360, 460), autour dudit premier axe, avant que ladite première surface d'articulation (136, 236, 336, 436) contacte ladite deuxième surface d'articulation (156, 256, 356, 456).

19. Dispositif d'arthroplastie de disque intervertébral selon la revendication 18, dans lequel ledit premier angle est dimensionné de sorte à permettre la rotation relative entre lesdits premier et deuxième éléments jusqu'à un troisième angle prédéterminé dans une direction allant vers lesdites extrémités antérieures, autour du premier axe, avant que ladite première surface d'articulation (136, 236, 336, 436) contacte ladite deuxième surface d'articulation (156, 256, 356, 456).

20. Dispositif d'arthroplastie de disque intervertébral selon la revendication 19, dans lequel ladite articulation à rotule et logement de rotule (126, 146, 226, 246, 326, 346, 426, 446) englobe une partie de rotule (146, 246, 346, 446) définie sur ladite première surface d'articulation (136, 236, 336, 436) dudit premier élément, le long dudit premier axe, et une partie de logement de rotule (126, 226, 326, 426) définie de manière correspondante dans ladite deuxième surface d'articulation (156, 256, 356, 456) dudit deuxième élément pour recevoir ladite partie de rotule.

21. Dispositif d'arthroplastie de disque intervertébral selon la revendication 20, dans lequel ladite partie de rotule (146, 246, 346, 446) a une section transversale circulaire le long de ladite première surface d'articulation.

22. Dispositif d'arthroplastie de disque intervertébral selon la revendication 15, dans lequel ladite articulation à rotule et logement de rotule (126, 146) est allongée et comprend une partie de rotule (146) définie sur ladite première surface d'articulation (136) dudit premier élément et allongée le long d'un premier axe parallèle à ladite ligne médiane transversale, et
une partie de logement de rotule (126) définie de manière correspondante dans ladite deuxième surface d'articulation (156) dudit deuxième élément pour recevoir ladite partie de rotule allongée (146) par l'intermédiaire d'un ajustement prédéterminé pour permettre une rotation relative prédéterminée entre lesdits premier et deuxième éléments autour d'un deuxième axe perpendiculaire auxdites première et deuxième surfaces d'articulation (136, 156) avant que ladite partie de rotule allongée (146) contact ladite partie de logement de rotule correspondante (126).

23. Dispositif d'arthroplastie de disque intervertébral selon la revendication 22, dans lequel lesdites parties de rotule et de logement de rotule allongées (226, 246) ont une section transversale en ellipse dans un plan parallèle à ladite première surface d'articulation.

24. Dispositif d'arthroplastie de disque intervertébral selon la revendication 22, dans lequel lesdites parties de rotule et de logement de rotule allongées (226, 246) ont une section transversale partiellement circulaire dans un plan parallèle à ladite première surface d'articulation et englobent des extrémités opposées ayant une forme partiellement cylindrique.

25. Dispositif d'arthroplastie de disque intervertébral selon la revendication 22, dans lequel ladite articulation à rotule et à logement de rotule allongée (326, 346) englobe plusieurs parties de rotule séparées et des parties de logement de rotule correspondantes.

26. Dispositif d'arthroplastie de disque intervertébral selon la revendication 25, dans lequel lesdites plusieurs parties de rotule et de logement de rotule (326, 346) englobent:
une première partie de rotule et de logement de rotule (326a, 346a); et
des deuxième (326b, 346b) et troisième (326c, 346c) parties de rotule et de logement de rotule espacées sur les côtés opposées de ladite première partie de rotule (346a) et situées à une distance égale de ladite première partie de rotule.

27. Dispositif d'arthroplastie de disque intervertébral selon la revendication 26, dans lequel lesdites première (346a), deuxième (346b) et troisième (346c) parties de rotule ont une section transversale circulaire dans un plan parallèle à ladite première surface d'articulation, ladite première partie de rotule (346a) ayant un rayon supérieur au rayon desdites deuxième (346b) et troisième (346c) parties de rotule.

28. Dispositif d'arthroplastie de disque intervertébral selon la revendication 27, dans lequel lesdites deuxième (346b) et troisième (346c) parties de rotule ont des rayons égaux.

29. Dispositif d'arthroplastie de disque intervertébral selon la revendication 1 ou 2, dans lequel les extrémités postérieure (360) et antérieure (358) dudit premier élément définissent une ligne médiane transversale entre elles, ladite ligne médiane étant située à une distance égale de ladite première extrémité postérieure et de ladite première extrémité antérieure;
un joint étant agencé entre lesdits premier et deuxième éléments pour permettre la rotation relative entre lesdits premier et deuxième éléments autour d'un premier axe parallèle à ladite ligne médiane transversale et un deuxième axe perpendiculaire audit premier axe, ladite articulation englobant:
une première (346a), deuxième (346b) et troisième (346c) parties de rotule définies dans ladite première surface d'articulation (336) dudit premier élément, lesdites parties de rotule ayant des sections transversales circulaires le long d'un axe parallèle à ladite ligne médiane transversale, ladite première partie de rotule (346a) ayant un rayon supérieur à celui desdites deuxième (346b) et troisième (346c) parties de rotule, lesdites deuxième et troisième parties de rotule ayant des rayons égaux et étant agencées sur les côtés opposés à ladite première partie de rotule, lesdites parties de rotule étant agencées radialement autour dudit premier axe; et
une partie de logement de rotule (326a, 326b, 326c) définie dans ladite deuxième surface d'articulation, pratiquement complémentaire desdites parties de rotule (346a, 346b, 346c) dans ladite première surface d'articulation (336) et configurée de sorte à recevoir lesdites parties de rotule;
lesdites surfaces d'engagement desdites première et deuxième coupelles d'insertion (70, 170) étant agencées de manière convexe autour dudit deuxième axe.
